# EUROPEAN PATENT SPECIFICATION

(11) **EP 3 603 608 B1**
(45) Date of publication and mention of the grant of the patent: **20.08.2025**
(21) Application number: 18774881.9
(22) Date of filing: 30.03.2018
(51) Int. Cl.: A61K 8/02, A61K 8/81, A61Q 19/00, A61K 8/04, A61K 8/26, A61Q 19/08, A61K 8/362

(54) **GEL SHEET**
GELFOLIE
FEUILLE DE GEL

(30) Priority: 31.03.2017 JP 2017072831
(43) Date of publication of application: 05.02.2020
(73) Proprietor: Sekisui Plastics Co., Ltd., Osaka-shi, Osaka 530-8565 (JP)
(72) Inventor: HATORI, Takaaki, Osaka-shi Osaka 530-8565 (JP)
(74) Representative: Müller-Boré & Partner Patentanwälte PartG mbB
(86) International application number: PCT/JP2018/013701
(87) International publication number: WO 2018/181930

(56) References cited:
- EP-A1- 1 043 023
- WO-A1-01/78678
- JP-A- 2004 083 413
- JP-A- 2005 095 331
- JP-A- 2006 335 961
- JP-A- 2010 202 609
- JP-A- 2010 202 609
- JP-A- 2010 222 320
- JP-A- 2015 098 554
- DATABASE GNPD [online] MINTEL; 19 August 2016 (2016-08-19), ANONYMOUS: "Jelly Mask", XP055754511, retrieved from www.gnpd.com Database accession no. 4399849
- ANONYMOUS: "HABA Jelly Mask ( 5 sheet ) | HABA USA Official", 21 January 2018 (2018-01-21), pages 1 - 8, XP055754757, Retrieved from the Internet <URL:https://web.archive.org/web/20180121024010/https://www.habaamerica.com/haba-jelly-mask.html> [retrieved on 20201127]
- DATABASE GNPD [online] MINTEL; 7 October 2016 (2016-10-07), ANONYMOUS: "Moisturising Night Eye Mask", XP055754516, retrieved from www.gnpd.com Database accession no. 4331093
- ANONYMOUS: "HABA Jelly Mask ( 5 sheet ) | HABA USA Official", 21 January 2018 (2018-01-21), pages 1 - 8, XP055754757, Retrieved from the Internet <URL:https://web.archive.org/web/20180121024010/https://www.habaamerica.com/haba-jelly-mask.html> [retrieved on 20201127]

## Description

### Technical Field

The present invention relates to a cosmetic mask gel sheet to be applied to the face, arms, legs, and the like.

### Background Art

Cosmetic mask gel sheets are external preparations comprising a hydrogel mask material containing a large amount of water. At the time of use, water containing a medicinal ingredient and the like, which is contained in gel, exudes to the surface of the gel, and the water and the like permeate through the skin to which the gel is applied, thereby achieving moisturizing effects and the like.

Patent Literature 1 discloses, as a conventional cosmetic mask gel sheet, a sheet-type mask preparation containing a water-soluble polymer, a polyalcohol, a moisturizing component, a cross-linking agent, a skin-care component, and water as essential components.

In addition, Patent Literature 2 discloses a sheet-type water-containing gel in which a synthetic polymer forming a network structure is impregnated with a solution containing at least water, a surfactant, and an oily ingredient and a liquid exudation promoter, the water-containing gel having a thickness of 1 mm and an amount of exuding liquid of 1.5 to 20 mg/cm² on its surface 72 hours after the formation of the water-containing gel.

MINTEL in entry 4399849 discloses a commercial Jelly Mask for the cheeks, based on polyacrylate, peg-20 and magnesium aluminometasilicate. It has air bubbles (https://www.gnpd.com/sinatra/ recordpage/4399849/).

### Citation List

### Patent Literature

Patent Literature 1: JP Patent Publication (Kokai) No. 8-188527 A (1996)
Patent Literature 2: JP Patent Publication (Kokai) No. 2003-183147 A

### Summary of Invention

### Technical Problem

Patent Literature 1 teaches that critical relative humidity levels of base components other than water are decreased by adding a polyalcohol such as a glycol, and a large amount of water is allowed to be released outside during use, thereby moisturizing the skin. In addition, a non-ionic water-soluble polymer is used as a liquid exudation promoter in the sheet-type water-containing gel disclosed in Patent Literature 2, and the non-ionic water-soluble polymer allows a solution containing medicinal ingredients and the like to exude. These conventional techniques increase the amount of exudation (the exudation amount) by allowing specific components to be contained, but still have room for improvement.

In consideration of the above-described circumstances, an object of the present invention is to provide a cosmetic mask gel sheet having a high exudation amount, by which a sufficient moisturizing feeling can be obtained as a result of penetration of water and the like through the skin to which the gel sheet is applied.

### Solution to Problem

As a result of intensive studies, the present inventors found that the above-described object can be achieved by forming air bubbles having a specific diameter in a gel sheet and controlling the number of the air bubbles. This has led to the completion of the invention. Specifically, the present invention is as defined in the appended set of claims and is summarized as follows.

(1) A cosmetic mask gel sheet containing at least water and a synthetic polymer forming a network structure by reacting with a cross-linking agent, wherein the gel sheet includes 10 to 100 air bubbles having a diameter of 0.1 mm to 1.0 mm in an area of 1 cm² in a plan view thereof while not including air bubbles having a diameter of 3.0 mm or more.
(2) The gel sheet according to (1), which includes 10 to 50 air bubbles having a diameter of 0.1 mm to 1.0 mm.
(3) The gel sheet according to (1) or (2), wherein the ratio of the number of air bubbles having a diameter of 0.1 mm to less than 0.5 mm and the number of air bubbles having a diameter of 0.5 to 1.0 mm is 1:2 to 15:1.
(4) The gel sheet according to any one of (1) to (3), which contains at least 1% by weight to 5% by weight of sodium polyacrylate as a synthetic polymer and 30% by weight to 90% by weight of water.
(5) The gel sheet according to any one of (1) to (4), wherein the average surface roughness Sa of arbitrary five regions having a size of 2 mm × 2 mm in an area of 1 cm² in a plan view of the gel sheet is 15 µm to 100 µm.
(6) The gel sheet according to any one of (1) to (5), wherein an intermediate base material comprising woven fabric or unwoven fabric is embedded along a plane direction of the gel sheet.
(7) The gel sheet according to (6), wherein the intermediate base material has a weight per unit area of 10 g/m² to 40 g/m².

### Advantageous Effects of Invention

According to the present invention, air bubbles form fine asperities on the surface of a gel sheet such that the specific surface area of the gel sheet increases, thereby increasing the exudation amount of water and the like from the surface. Therefore, the use of the gel sheet of the present invention allows more water to penetrate into the skin. As a result, an excellent moisturizing feeling can be obtained.

### Brief Description of Drawings

[Figure 1] Figure 1 schematically illustrates a cross-section of a gel sheet in one embodiment according to the present invention.
[Figure 2] Figure 2 schematically illustrates a cross-section of a gel sheet in another embodiment according to the present invention.

### Description of Embodiments

The present invention will be described in detail below.

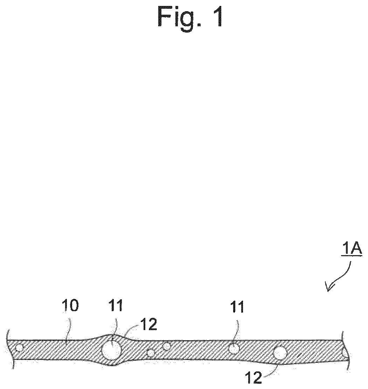

Figure 1 is a cross-sectional view of a gel sheet in one embodiment of the present invention. A gel sheet 1A in the figure is composed of a gel material 10 containing at least water and a synthetic polymer forming a network structure by reacting with a cross-linking agent. In addition, the gel sheet 1A in this embodiment is characterized in that it includes 10 to 100 air bubbles 11 having a diameter of 0.1 mm to 1.0 mm in an area of 1 cm² in a plan view thereof. Since the gel sheet 1A include the air bubbles 11, fine asperities 12 are formed on each surface of the gel sheet 1A along the air bubbles 11. Accordingly, the specific surface area of the gel sheet 1A increases, thereby increasing the exudation amount of water or the like exuding from the surface of the gel sheet. Here, the diameter of air bubbles 11 is the average value of the major axis and minor axis measured in a plan view when the surface of the gel sheet 1A is microscopically observed.

When the number of air bubbles 11 is less than 10, the effect of increasing the exudation amount cannot be obtained. On the other hand, when it is excessively increased, adhesion of the gel sheet 1A might be reduced. Therefore, the number of air bubbles 11 is appropriately predetermined in consideration of a balance between advantageous and disadvantageous effects. The number of air bubbles having a diameter of 0.1 mm to 1.0 mm is preferably 10 to 80 and particularly preferably 10 to 50. In addition, the gel sheet 1A does not include air bubbles having a diameter of 3 mm or more. This is because air bubbles having a diameter of 3 mm or more cause an excess in surface asperities, resulting in reduced adhesion of the gel sheet 1A.

In addition, when the diameter of air bubbles 11 is biased to a specific value, surface asperities are averaged, which may reduce the effect of increasing the specific surface area of the gel sheet 1A. It is therefore preferable that air bubbles having a large diameter and air bubbles having a small diameter exist in a mixed manner. Specifically, the ratio of the number of air bubbles having a diameter of 0.1 mm to less than 0.5 mm and the number of air bubbles having a diameter of 0.5 to 1.0 mm is in a range of 1:2 to 15:1.

In addition, when the value of surface roughness of the gel sheet 1A is excessively large, it causes an excess in surface asperities, resulting in reduced adhesion of the gel sheet 1A. Therefore, the value of surface roughness is in a specified range. Specifically, in a case in which the average surface roughness Sa of arbitrary five regions having a size of 2 mm × 2 mm in an area of 1 cm² in a plan view of the gel sheet 1A is measured, the Sa value is in a range of 15 µm to 100 µm and more preferably 15 µm to 80 µm. When the Sa value is within the above range, asperities are appropriately formed on the surface of the gel sheet 1A such that the exudation amount of water and the like can be increased.

The thickness of the gel sheet 1A can be appropriately set in accordance with the intended use of the gel sheet 1A and the composition of the gel material 10. In order to ensure adhesion, transparency, and strength, the thickness is preferably 0.1 mm to 3.0 mm. When the thickness is less than 0.1 mm, the gel sheet 1A cannot include air bubbles 11 and has a decreased strength in some cases. When the thickness is more than 3 mm, the weight of the gel sheet 1A itself excessively increases, resulting in lack of adhesion in some cases. In a case in which the entire thickness of the gel sheet 1A is not large, the thickness of the gel sheet 1A may be partially reduced to 6.0 mm.

Next, materials constituting the gel sheet 1A will be described.

### (Synthetic polymer)

A synthetic polymer used herein is not particularly limited as long as it forms a network structure and is capable of forming gel with water contained therein. Synthetic polymers that can be applied to the skin in the field of cosmetic masks are applicable. In particular, synthetic polymers having affinity with water are used. Of these, synthetic polymers having an anionic functional group are preferably used. The synthetic polymers used in the present invention are polymers of polymerizable unsaturated monomers which are acrylic acid or methacrylic acid and salts thereof (e.g., sodium salts, potassium salts, and triethanolamine salts). Sodium polyacrylate, which is a hydrophilic polymer, is preferably used. The content of a synthetic polymer is preferably 0.5% by weight to 6% by weight in the gel sheet 1A. In particular, in a case in which sodium polyacrylate is used, the content thereof in the gel sheet 1A is preferably 1% by weight to 5% by weight.

A non-crosslinked synthetic polymer may be partially crosslinked unless it prevents the production of gel sheet 1A. In addition, in consideration of handleability at the time of manufacturing, it is preferable to use a non-crosslinked synthetic polymer having a weight-average molecular weight of 100,000 to 5,000,000.

### (Water)

When the water content in the gel sheet 1A is excessively small, the water content relative to the equilibrium water content of the gel sheet 1A decreases and hygroscopicity of the gel sheet 1A increases, which may cause the gel sheet 1A to alter (e.g., swelling) over time. On the other hand, when the water content is excessively large, the water content relative to the equilibrium water content of the gel sheet 1A increases, which may cause shrinkage or changes in physical properties of the gel sheet 1A due to drying. Therefore, the water content is appropriately set in consideration of these facts. Specifically, the water content in gel sheet 1A is set to preferably 30% by weight to 90% by weight.

### (Cross-linking agent)

The network structure of a synthetic polymer is obtained by cross-linking of the synthetic polymer. In other words, the network structure can be formed by adding a cross-linking agent to a non-crosslinked synthetic polymer and, if necessary, heating the polymer. Examples of a cross-linking agent include aluminum hydroxide, potassium alum, aluminum sulfate, aluminum glycinate, aluminum acetate, aluminum oxide, aluminum metasilicate, magnesium chloride, calcium hydroxide, calcium carbonate, magnesium metasilicate aluminate, polyethylenimine, polyethylene glycol diglycidyl ether, glycerin triglycidyl ether, and triglycidyl isocyanurate. The cross-linking agent content in the gel sheet 1A is set to preferably 0.1% by weight to 5% by weight.

### (pH adjuster)

In addition, in order to adjust an optimal pH to allow a cross-linking agent to act, α-hydroxy acids such as tartaric acid, lactic acid, and glycolic acid, various organic acids such as citric acid, inorganic acids such as hydrochloric acid, and the like may be used as a pH adjuster (acid catalyst). The pH adjuster content in the gel sheet 1A is set to preferably 0.1% by weight to 10% by weight.

### (Exudation promoter)

A exudation promoter is a component used for allowing a solution such as water in the gel sheet 1A to effectively exude to the surface of the gel sheet. It is also referred to as "liquid exudation promoter." The exudation promoter is a non-ionic water-soluble polymer having a structure that is hydrophilic but has hydrophobicity to some extent so as to allow water in the gel sheet 1A to be effectively discharged or an electrolyte capable of allowing a solution in the gel sheet 1A to exude to the surface of the gel sheet by exerting the effect of tightening the network structure of the gel sheet 1A.

Examples of a non-ionic water-soluble polymer include polymers of glycols such as polyethylene glycol, polypropylene glycol, and polyglycerin and polyalcohols. These can be used alone or in mixture of two or more types thereof. The non-ionic water-soluble polymer content in the gel sheet 1A is preferably 0.1% by weight to 20% by weight or less and more preferably 1% by weight to 10% by weight.

In particular, assuming that the hygroscopicity of glycerin is 100, it is preferable to use a non-ionic water-soluble polymer having a specific hygroscopicity of 2 to 55. When the specific hygroscopicity is excessively low, the amount of liquid exudation to the surface of the gel sheet 1A excessively increase due to insufficient water-retaining capacity, which causes a sticky feeling or poor compatibility with a synthetic polymer. This may make it difficult to form a gel sheet 1A. In addition, when the specific hygroscopicity is excessively high, a non-ionic water-soluble polymer has excessively high water-retaining ability, which makes it difficult for a solution in the gel sheet 1A to exude. This may result in lack of a moist feeling and moisturizing effects. The specific hygroscopicity is more preferably 20 to 45.

Any electrolyte can be used herein as long as it is highly soluble in water and has been actually used in cosmetics and the like. However, neutral salts of strong acids or alkalis are preferably used because of, for example, fewer fluctuations in solubility and pH. Examples of such electrolytes include sodium chloride, potassium chloride, sodium sulfate, and potassium sulfate. The electrolyte content in the gel sheet 1A is preferably 0.1% by weight to 20% by weight and more preferably 0.2% by weight to 5% by weight. Either a non-ionic water-soluble polymer or an electrolyte may be blended or both of them may be blended in combination.

### (Moisturizer)

Examples of a moisturizer include: glycols such as ethylene glycol, diethylene glycol, triethylene glycol, propylene glycol, dipropylene glycol, glycerin, diglycerin, isopropylene glycol, 1,3-butylene glycol, sorbitol, marbitol, trehalose, raffinose, xylitol, mannitol, hyaluronic acid, and salts thereof, various derivatives such as trehalose and raffinose, trimethylglycine, cyclodextrin, hyaluronic acid, and salts thereof; polyalcohols; and polysaccharides. These can be used alone or in mixture of two or more types thereof. The moisturizer content in the gel sheet 1A is preferably 1% by weight to 30% by weight.

### (Medicinal ingredient)

The gel sheet 1A may include a medicinal ingredient which is conventionally used for pharmaceutical products, quasi-drugs, cosmetics, hygiene materials, and the like if necessary. Examples of a medicinal ingredient include avocado extract, *Hydrangea serrata* leaf extract, *Echinacea angustifolia* leaf extract, collagen, lemon extract, and rose extract.

Examples thereof also include: biopolymers such as collagen, chitin, and chitosan; moisturizing components such as amino acids, urea, betaine, whey, and trimethyl glycine; oily ingredients such as squalane, ceramide, and phospholipid; anti-inflammatory agents such as glycyrrhizinic acid and β-glycyrrhetinic acid; vitamins such as vitamins A, B2, B6, D, and E and nicotinic acid amide; antioxidants such as tocopherol; wound healing agents such as retinol and retinol derivatives.

The blending amounts of these medicinal ingredients cannot be uniformly specified because the amounts of ingredients differ depending on types of medicinal ingredients. However, usually, the content of a medicinal ingredient in the gel sheet 1A is preferably 0.001% by weight to 80% by weight and more preferably 0.05% by weight to 30% by weight.

In addition, the gel sheet 1A may include a thickener if necessary. When components constituting the gel sheet 1A are mixed by stirring or the like to prepare a mixture, a thickener functions to increase viscosity of the mixture and harden the mixture while preventing air bubbles 11 formed by, for example, whisking the mixture from escaping outside the gel sheet 1A so as to allow the air bubbles remain inside the gel sheet 1A. Examples of such a thickener include agar, gelatin, carrageenan, xanthan gum, celluloses (e.g., carboxymethylcellulose (CMC), methylcellulose (MC), and hydroxypropyl cellulose (HPC)), polyacrylic acid or a salt thereof, polymethacrylic acid or a salt thereof, polyvinyl alcohol, polyvinyl pyrrolidone, alginic acid or a salt thereof, polyacrylamide, monomers thereof, and copolymers of such monomers and other monomers. In addition, the thickener content in the gel sheet 1A is, but is not limited to, preferably 0.1% by weight to 1% by weight.

Further, the gel sheet 1A may contain other additives if necessary. Examples of other additives include a preservative, an antirust agent, an antifungal agent, an antioxidant, an antifoamer, a stabilizer, a surfactant, and a colorant. The contents of those other additives differ depending on types of additives. However, the total content of such additives in the gel sheet 1A is preferably 0.01% by weight to 5% by weight.

### (Production method)

The gel sheet 1A can be produced by a usual method for producing a gel sheet. For example, a gel sheet 1A can be obtained by mixing a synthetic polymer, water, a cross-linking agent, a moisturizer, a exudation promoter, and a preservative as starting materials by stirring, further adding a pH adjuster and water to the obtained mixture, and if necessary, heating the resulting mixture to induce a reaction. Air bubbles formed at such time are controlled such that the obtained gel sheet includes 10 to 100 air bubbles having a diameter of 0.1 mm to 1.0 mm in an area of 1 cm² in a plan view thereof while not including air bubbles having a diameter of 3.0 mm or more. Specifically, for example, air bubbles can be controlled based on the rotational speed during mixing of the components by stirring and the aging time and temperature for the stirred mixture. The optimal rotational speed and aging time and temperature for the mixture are not particularly limited because they may differ depending on the amounts of components, the size of a container, and the like. Alternatively, it is also possible to produce a gel sheet 1A by blending in a thickener and hardening the mixture while allowing the formed air bubbles to remain therein. In addition, it is preferable to adjust the pH of the gel composition to 4 to 7 by adding a pH adjuster (acid catalyst) so as to induce a reaction.

If necessary, it is possible to provide a base film on one side of the gel sheet 1A and provide a top film on the side opposite to the side on which the base film is provided.

Examples of the above-described base film that can be used herein include resin films made of resins such as polyester, polyolefin, polystyrene, and polyurethane, paper, and paper obtained by laminating the resin films.

Basically, the material of the base film can be used as the material of the top film. However, the top film is not limited thereto.

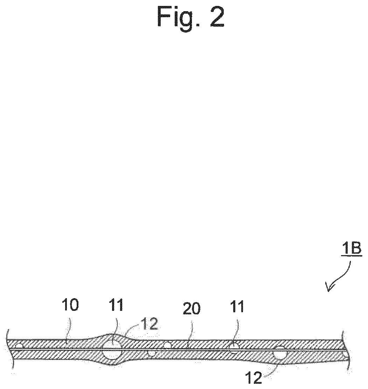

Figure 2 is a cross-sectional view of a gel sheet in another embodiment of the present invention. In this embodiment, in order to improve tear strength and handleability of a gel sheet 1B without impairing transparency of the gel sheet 1B, an intermediate base material 20 comprising woven fabric or unwoven fabric is embedded along a plane direction of the gel sheet 1B. Materials that can be used for unwoven fabric and woven fabric include natural fibers such as cellulose, silk, and hemp, synthetic fibers such as polyester, nylon, rayon, polyethylene, polypropylene, and polyurethane, and mixtures thereof. If necessary, a binder may be used, and such fabric may be colored.

Examples of a method for producing the unwoven fabric include, but are not particularly limited to, a dry method or wet method, a spunbond method, a melt-blown method, an air-laid method, a chemical bond method, a thermal bond method, a needle punch method, and a water entangling method. It is preferable to adopt a production method depending on the weight per unit area or material such that there is no disparity in weight per unit area for controlling the position of the intermediate base material 20. Woven fabric is also not particularly limited to, for example, plain weave, tricot, or raschel fabric, and can be appropriately selected.

In addition, the weight per unit area of the woven fabric or unwoven fabric described above is not particularly limited as long as given properties of the intermediate base material 20 can be obtained based on the weight per unit area. However, for example, it is preferably 10 g/m² to 40 g/m² and more preferably 10 g/m² to 28 g/m². When the weight per unit area of the above-described woven fabric or unwoven fabric is excessively small, it may make it difficult to, for example, reinforce the gel sheet 1B, or disparity in weight per unit area increases, which may cause liquid permeability to change by site during production of the place gel sheet 1B, causing the position of the intermediate base material 20 to change. In addition, when the weight per unit area is excessively large, the intermediate base material 20 becomes hard, and the ability of the gel sheet 1B to, for example, fit to the skin may be impaired. Therefore, the weight per unit area is appropriately determined in consideration of a favorable balance of such properties.

When the thickness of the intermediate base material 20 is excessively increased, liquid permeability might deteriorate. On the other hand, when the thickness is excessively decreased, it may make it difficult to, for example, reinforce the gel sheet 1B or it may cause the position of the intermediate base material 20 to change as in the case of the excessively decreased weight per unit area. Therefore, the thickness is appropriately determined in consideration of these factors. The thickness is preferably 0.05 mm to 2.0 mm, more preferably 0.05 mm to 0.5 mm, and particularly preferably 0.08 mm to 0.3 mm.

A method of producing the gel sheet 1B is not particularly limited because conditions differ depending on the composition of the gel material 10 and the material, thickness, and the like of the intermediate base material 20. Examples of the method that can be appropriately adopted are: a method comprising holding an intermediate base material 20 in the air while applying a constant tension thereto, pouring a gel composition obtained by mixing components thereof on the upper and lower sides of the intermediate base material 20, and cross-linking/hardening the gel composition, thereby forming the gel composition into a sheet; a method comprising preparing two smooth-surface gel sheets and sandwiching an intermediate base material 20, which is held while a constant tension is applied thereto, with the gel sheets, thereby obtaining a composite thereof; and a method comprising preparing a smooth-surface gel sheet, placing an intermediate base material 20 on the gel sheet while a constant tension is applied to the intermediate base material 20, pouring a gel composition obtained by mixing components thereof on the intermediate base material 20, and cross-linking/hardening the gel composition.

The other components of the configuration of the gel sheet 1B are the same as those in the embodiment of the gel sheet 1A depicted in Figure 1 as described above.

### Examples

The present invention will be described in more detail below based on the Examples and Comparative Examples. However, the present invention is not limited to these examples.

### (Examples 1 to 5 and Comparative Examples 1 to 6)

Water (75.7% by weight), sodium polyacrylate (synthetic polymer: 3.5% by weight), magnesium metasilicate aluminate (cross-linking agent: 0.8% by weight), dipropylene glycol (moisturizer: 10% by weight), polyethylene glycol 1000 (exudation promoter: 3.5% by weight), and phenoxyethanol (preservative:0.5% by weight) as components (470 ml in total) were sufficiently kneaded in a plastic container (500 ml) and stirred using, as a stirrer, THREE-ONE MOTOR BL 3000 manufactured by Shinto Scientific Co., Ltd., thereby preparing liquid mixture A. The rotational speed for stirring is described in Table 1. After stirring, each mixture was aged in accordance with the corresponding aging time and temperature described in Table 1.

Next, tartaric acid (pH adjuster: 1.8% by weight) was dissolved in 4.2% by weight of water in a different container, and the aqueous solution thereof (liquid B) was added to and mixed with the liquid A, thereby preparing a gel composition having a pH of 5 to 5.5. Subsequently, a 100-µm PET as a film base film was coated with the gel composition, and a woven nylon fabric (tricot half) having a weight per unit area of 17 g/m² as an intermediate base material was immersed in the gel composition such that the fabric was placed at the approximate middle position in the thickness direction of the gel composition. Then, a 38-µm PET film as a top film was layered over the gel composition such that the total thickness of the gel sheet (including the intermediate base material) comprising the top film and the gel composition and the base film became 1.0 mm, and the layered product was left for 24 hours. Thus, the gel composition was reacted and hardened, thereby producing a gel sheet. A period of time during which coating was completed after the addition of the liquid B was set to 3 minutes.

### (Example 6)

Water (73.65% by weight), sodium polyacrylate (synthetic polymer: 3.5% by weight), magnesium metasilicate aluminate (cross-linking agent: 0.8% by weight), dipropylene glycol (moisturizer: 10% by weight), polyethylene glycol 1000 (exudation promoter: 3.5% by weight), phenoxyethanol (preservative: 0.5% by weight), squalane (medicinal ingredient) (oil component: 2.0% by weight), and polyglyceryl laurate (emulsifier: 0.05% by weight) as components (470 ml in total) were sufficiently stirred and kneaded in a plastic container (500 ml) and further stirred using, as a stirrer, THREE-ONE MOTOR BL 3000 manufactured by Shinto Scientific Co., Ltd., thereby preparing liquid mixture A. The rotational speed for stirring is described in Table 1. After stirring, each mixture was aged in accordance with the corresponding aging time and temperature described in Table 1. Thereafter, gel sheets were produced in the same manner as in Example 1.

### (Example 7)

Water (76.2% by weight), sodium polyacrylate (synthetic polymer: 3.0% by weight), magnesium metasilicate aluminate (cross-linking agent: 0.8% by weight), dipropylene glycol (moisturizer: 10% by weight), polyethylene glycol 1000 (exudation promoter: 3.5% by weight), and phenoxyethanol (preservative:0.5% by weight) as components (470 ml in total) were sufficiently stirred and kneaded in a plastic container (500 ml) and further stirred using, as a stirrer, THREE-ONE MOTOR BL 3000 manufactured by Shinto Scientific Co., Ltd., thereby preparing liquid mixture A. The rotational speed for stirring is described in Table 1. After stirring, each mixture was aged in accordance with the corresponding aging time and temperature described in Table 1. Thereafter, gel sheets were produced in the same manner as in Example 1.

Regarding each obtained gel sheet, the number of air bubbles having a diameter of 0.1 mm to 1.0 mm and the presence or absence of air bubbles having a diameter of 3.0 mm or more in an area of 1 cm² in a plan view were evaluated using a Digital Microscope VHX-700F manufactured by KEYENCE CORPORATION. In addition, the ratio of the number of air bubbles having a diameter of 0.1 mm to less than 0.5 mm and the number of air bubbles having diameter of 0.5 mm to 1.0 mm (a:b) was determined. Table 1 list the measurement results.

In addition, regarding each obtained gel sheet, the average surface roughness Sa of arbitrary five regions having a size of 2 mm × 2 mm in an area of 1 cm² in a plan view was measured using a 3D Laser Scanning Microscope VK-X1000 (measurement magnification: × 100) manufactured by KEYENCE CORPORATION. Table 1 list the measurement results. It was difficult to measure the average surface roughness Sa in Comparative Examples 3 and 4 due to the presence of coarse air bubbles.

Further, regarding obtained gel sheet, the exudation amount, adhesion, feeling of use, and appearance were evaluated in terms of quality. The evaluation was made by subjects based on the following two-step and three-step criteria, and the evaluation results were obtained based on the total score of each item.

The exudation amount was evaluated by applying each prepared gel sheet in a state of having a lifted portion to the cheek of each of the five subjects. Two points were given in a case in which a moisture feeling remained after removal of the gel sheet from the skin, one point was given in a case in which a moisture feeling slightly remained after removal of the gel sheet from the skin, and zero point was given in a case in which no moisture feeling remained gel sheet after removal of the gel sheet from the skin. A gel sheet with seven or more points in total was rated as "o" (favorable), a gel sheet with four to six points in total was rated as "Δ" (acceptable), and a gel sheet with three or less points in total was rated as "×" (unfavorable).

In addition, the adhesion was evaluated by applying each prepared gel sheet in a state of having a lifted portion to the cheek of each of the five subjects. Two points were given in a case in which a feeling that the gel sheet fits to the skin was sufficiently felt while the gel sheet was applied to the face, one point was given in a case in which a feeling that the gel sheet fits to the skin was slightly felt while the gel sheet was applied to the face, and zero point was given in a case in which a feeling that the gel sheet fits to the skin was not felt while the gel sheet was applied to the face. A gel sheet with seven or more points in total was rated as "o" (favorable), a gel sheet with four to six points in total was rated as "Δ" (acceptable), and a gel sheet with three or less points in total was rated as "×" (unfavorable).

Further, the feeling of use was evaluated by applying each prepared gel sheet in a state of having a lifted portion to the cheek of each of the five subjects. One point was given in a case in which a moisturizing feeling was likely to be felt when applying the gel sheet to the skin, and zero point was given in a case in which a moisturizing feeling was unlikely to be felt when applying the gel sheet to the skin. A gel sheet with three or more points in total was rated as "∘" (favorable) and a gel sheet with two or less points in total was rated as "×" (unfavorable).

Furthermore, the appearance was evaluated by visual inspection of five subjects. One point was given in a case in which the appearance of the sheet looked favorable before application to the skin, and zero point was given in a case in which the appearance of the sheet looked unfavorable before application to the skin (a feeling of a foreign body or a feeling of a reject due to dents). A gel sheet with three or more points in total was rated as "o" (favorable) and a gel sheet with two or less points in total was rated as "×" (unfavorable).

Table 1 list the evaluation results.

**[Table 1]**

| | Example | | | | | | | Comparative Example | | | | | |
|---|---|---|---|---|---|---|---|---|---|---|---|---|---|
| | 1 | 2 | 3 | 4 | 5 | 6 | 7 | 1 | 2 | 3 | 4 | 5 | 6 |
| Presence or absence of medicinal ingredient | Absent | Absent | Absent | Absent | Absent | Present | Absent | Absent | Absent | Absent | Absent | Absent | Absent |
| Rotational speed [rpm] | 150 | 100 | 150 | 150 | 150 | 150 | 150 | 150 | 150 | 150 | 150 | 150 | 150 |
| Aging time [hr] | 24 | 24 | 18 | 6 | 3 | 24 | 24 | 48 | 36 | 18 | 6 | 1.5 | 0 |
| Temperature of mixture [°C] | 20 | 10 | 20 | 20 | 20 | 20 | 20 | 20 | 20 | 5 | 5 | 20 | 20 |
| Number of 0.1- to 1.0-mm air bubbles | 14 | 15 | 21 | 73 | 98 | 30 | 27 | 0 | 5 | 16 | 42 | 123 | 141 |
| Ratio of diameter of air bubbles (a:b) | 8:6 | 6:9 | 12:9 | 68:5 | 86:12 | 18:12 | 17:10 | - | 3:2 | 9:7 | 29:13 | 103:20 | 114:27 |
| Ratio of diameter of air bubbles (a/b) | 1.33 | 0.67 | 1.33 | 13.60 | 7.17 | 1.50 | 1.70 | - | 1.50 | 1.29 | 2.23 | 5.15 | 4.22 |
| Presence or absence of 3.0-mm or more air bubbles (number of air bubbles in parentheses) | Absent | Absent | Absent | Absent | Absent | Absent | Absent | Absent | Absent | Present (3) | Present (5) | Absent | Absent |
| Average surface roughness Sa [µm] | 18.6 | 18.9 | 21.8 | 47.6 | 75.3 | 26.8 | 20.5 | 5.3 | 14.6 | - | - | 103.2 | 108.5 |
| Exudation amount | ○ | ○ | ○ | ○ | ○ | ○ | ○ | × | × | ○ | ○ | Δ | Δ |
| Adhesion | ○ | ○ | ○ | ○ | ○ | ○ | ○ | ○ | ○ | Δ | Δ | × | × |
| Feeling of use | ○ | ○ | ○ | ○ | ○ | ○ | ○ | ○ | ○ | × | × | × | × |
| Appearance | ○ | ○ | ○ | ○ | ○ | ○ | ○ | ○ | ○ | × | × | × | × |

As listed in Table 1, the exudation amount was sufficiently large in the gel sheets in Examples 1 to 7, each of which included 10 to 100 air bubbles having a diameter of 0.1 mm to 1.0 mm in an area of 1 cm² in a plan view. Favorable results were obtained for each of the evaluation items of adhesion, feeling of use, and appearance. Meanwhile, the exudation amount was insufficient in the gel sheets in Comparative Examples 1 and 2, each of which included a small number of air bubbles. In addition, the gel sheets including air bubbles having a diameter of 3.0 mm or more in Comparative Examples 3 and 4 were disadvantageous in that the appearance looked unfavorable and problematic in that it was hard to feel a moisturizing feeling in terms of the feeling of use. Further, the gel sheets including more than 100 air bubbles in Comparative Examples 5 and 6 were problematic regarding the exudation amount because there were many voids in the gel, which conversely gave a feeling that the exudation amount was insufficient. The gel sheets were also problematic in that they were unfavorable in terms of the appearance and feeling of use.

In addition, the gel sheets in which the average surface roughness Sa of arbitrary five regions having a size of 2 mm × 2 mm was 15 µm to 100 µm in an area of 1 cm² in a plan view in Examples 1 to 7 were advantageous in that the exudation amount was sufficient and a moisture feeling remained even after removal of the gel sheets from the skin. Meanwhile, the gel sheets having an average surface roughness Sa of less than 15 µm in Comparative Examples 1 and 2 were considered to have insufficient amounts of exudation because of small specific surface areas due to lack of surface asperities. The gel sheets having an average surface roughness Sa of more than 100 µm in Comparative Examples 5 and 6 were problematic in terms of the adhesion and the like.

### Reference Signs List

1A, 1B: Gel sheet
10: Gel material
11: Air bubbles
12: Asperities
20: Intermediate base material

## Claims

1. A cosmetic mask gel sheet containing at least water and a synthetic polymer forming a network structure by reacting with a cross-linking agent,
wherein the synthetic polymer is a polymer of a polymerizable unsaturated monomer which is acrylic acid or methacrylic acid, or salts thereof;
wherein the gel sheet further contains a non-ionic water-soluble polymer or an electrolyte as an exudation promoter,
wherein the gel sheet includes 10 to 100 air bubbles having a diameter of 0.1 mm to 1.0 mm in an area of 1 cm² in a plan view thereof while not including air bubbles having a diameter of 3.0 mm or more,
wherein the average surface roughness Sa of arbitrary five regions having a size of 2 mm × 2 mm in an area of 1 cm² in a plan view of the gel sheet, which is measured using a 3D Laser Scanning Microscope VK-X1000 (measurement magnification: x 100) manufactured by KEYENCE CORPORATION, is 15 µm to 100 µm, and
wherein the ratio of the number of air bubbles having a diameter of 0.1 mm to less than 0.5 mm and the number of air bubbles having a diameter of 0.5 to 1.0 mm is 1:2 to 15:1.

2. The gel sheet according to claim 1, which includes 10 to 50 air bubbles having a diameter of 0.1 mm to 1.0 mm.

3. The gel sheet according to claim 1 or 2, which contains at least 1% by weight to 5% by weight of sodium polyacrylate as a synthetic polymer and 30% by weight to 90% by weight of water.

4. The gel sheet according to any one of claims 1 to 3, wherein an intermediate base material comprising woven fabric or unwoven fabric is embedded along a plane direction of the gel sheet.

5. The gel sheet according to claim 4, wherein the intermediate base material has a weight per unit area of 10 g/m² to 40 g/m².

## Patentansprüche

1. Gelfolie für kosmetische Maske, die zumindest Wasser und ein synthetisches Polymer enthält, das durch Reaktion mit einem Vernetzungsmittel eine Netzstruktur bildet,
wobei es sich bei dem synthetischen Polymer um ein Polymer eines polymerisierbaren ungesättigten Monomers handelt, das Acrylsäure oder Methacrylsäure, oder Salze davon ist;
wobei die Gelfolie ferner ein nichtionisches wasserlösliches Polymer oder ein Elektrolyt als ein Exsudations-Promotor enthält,
wobei die Gelfolie 10 bis 100 Luftblasen einschließt, die einen Durchmesser von 0,1 mm bis 1,0 mm auf einer Fläche von 1 cm² in einer Draufsicht davon aufweisen, wobei keine Luftblasen mit einem Durchmesser von 3,0 mm oder mehr eingeschlossen sind,
wobei die mittlere Oberflächenrauigkeit Sa von beliebigen fünf Regionen mit einer Größe von 2 mm x 2 mm auf einer Fläche von 1 cm² in einer Draufsicht auf die Gelfolie, unter Verwendung eines 3D Laserscanning-Mikroscops VK-X1000 (Vergrößerung der Messung: x 100), hergestellt durch KEYENCE CORPORATION, 15 µm bis 100 µm beträgt, und wobei das Verhältnis zwischen der Anzahl der Luftblasen mit einem Durchmesser von 0,1 mm bis weniger als 0,5 mm und der Anzahl der Luftblasen mit einem Durchmesser von 0,5 bis 1,0 mm 1:2 bis 15:1 beträgt.

2. Gelfolie nach Anspruch 1, die 10 bis 50 Luftblasen enthält, die einen Durchmesser von 0,1 mm bis 1,0 mm aufweisen.

3. Gelfolie nach Anspruch 1 oder 2, die zumindest 1 Gew.-% bis 5 Gew.-% Natriumpolyacrylat als ein synthetisches Polymer und 30 Gew.-% bis 90 Gew.-% Wasser enthält.

4. Gelfolie nach einem der Ansprüche 1 bis 3, wobei ein Zwischen-Grundmaterial, das gewebte Stoffe oder nicht gewebte Stoffe umfasst, entlang einer Ebenenrichtung der Gelfolie eingebettet wird.

5. Gelfolie nach Anspruch 4, wobei das Zwischen-Grundmaterial ein Gewicht pro Flächeneinheit von 10 g/m² bis 40 g/m² aufweist.

## Revendications

1. Feuille de gel pour masque cosmétique contenant au moins de l'eau et un polymère synthétique formant une structure de réseau par réaction avec un agent de réticulation,
le polymère synthétique étant un polymère d'un monomère insaturé polymérisable qui est l'acide acrylique ou l'acide méthacrylique ou des sels de ceux-ci ;
la feuille de gel contenant en outre un polymère hydrosoluble non ionique ou un électrolyte en tant que promoteur d'exsudation,
la feuille de gel renfermant 10 à 100 bulles d'air ayant un diamètre de 0,1 mm à 1,0 mm dans une surface de 1 cm² dans une vue en plan de celle-ci, sans renfermer de bulles d'air ayant un diamètre supérieur ou égal à 3,0 mm,
la rugosité de surface moyenne Sa de cinq zones arbitraires ayant une taille de 2 mm x 2 mm dans une surface de 1 cm² dans une vue en plan de la feuille de gel, qui est mesurée à l'aide d'un microscope à balayage laser 3D VK-X1000 (grossissement de mesure : x100) fabriqué par KEYENCE CORPORATION, étant de 15 µm à 100 µm et
le rapport du nombre de bulles d'air ayant un diamètre de 0,1 mm à moins de 0,5 mm et du nombre de bulles d'air ayant un diamètre de 0,5 à 1,0 mm étant de 1:2 à 15:1.

2. Feuille de gel selon la revendication 1, qui renferme 10 à 50 bulles d'air ayant un diamètre de 0,1 mm à 1,0 mm.

3. Feuille de gel selon la revendication 1 ou 2, qui contient au moins 1 % en poids à 5 % en poids de polyacrylate de sodium en tant que polymère synthétique et 30 % en poids à 90 % en poids d'eau.

4. Feuille de gel selon l'une quelconque des revendications 1 à 3, un matériau de base intermédiaire comprenant une étoffe tissée ou une étoffe non tissée étant incorporé le long d'une direction du plan de la feuille de gel.

5. Feuille de gel selon la revendication 4, le matériau de base intermédiaire ayant un poids par unité de surface de 10 g/m² à 40 g/m².
